# EUROPÄISCHE PATENTSCHRIFT

(11) **EP 3 698 669 B1**
(45) Veröffentlichungstag und Bekanntmachung des Hinweises auf die Patenterteilung: **15.11.2023**
(21) Anmeldenummer: 20158706.0
(22) Anmeldetag: 21.02.2020
(51) Int. Cl.: A45D 29/05, A61B 17/54, A45D 29/14, A61B 17/00

(54) **BEHANDLUNGSGERÄT UND BEHANDLUNGSSYSTEM**
TREATMENT DEVICE AND TREATMENT SYSTEM
APPAREIL DE TRAITEMENT ET SYSTÈME DE TRAITEMENT

(30) Priorität: 21.02.2019 DE 102019104494
(43) Veröffentlichungstag der Anmeldung: 26.08.2020
(73) Patentinhaber: Endlicher, Martin, 83075 Bad Feilnbach (DE)
(72) Erfinder: Endlicher, Martin, 83075 Bad Feilnbach (DE)
(74) Vertreter: Leonhard, Frank Reimund

(56) Entgegenhaltungen:
- WO-A1-2008/147134
- BR-U2-202012 007 242
- DE-U1- 29 710 333
- DE-U1- 29 805 307
- US-A1- 2013 092 182
- US-A1- 2015 201 969
- US-B1- 8 545 516

## Beschreibung

Die Erfindung betrifft ein Behandlungssystem mit einem Behandlungsgerät.

Aus dem Stand der Technik sind verschiedene Behandlungsgeräte zur Behandlung des menschlichen Fußes bekannt, insbesondere solche, die das Abschleifen von Hornhaut an den Füßen ermöglichen und erleichtern sollen, siehe beispielsweise das Dokument US 2015/201969 A1. Hornhaut an den Füßen kann beispielsweise durch manuelles Abschleifen mit Hilfe eines Bimssteins (zumindest teilweise) entfernt werden.

Alternativ dazu gibt es handgehaltene Geräte, die ein durch einen Motor angetriebenes Schleifelement zum Entfernen der Hornhaut umfassen. Durch das integrieren eines motorisierten Schleifelements im handgehaltenen Gerät wird dem Benutzer die manuelle Abschleifbewegung erspart, wodurch der Bedienungskomfort erhöht wird.

Dabei muss der Benutzer jeweils das in der Hand gehaltene Gerät zum Fuß (oder den Fuß zum Gerät) hinführen. Die Körperhaltungen, die notwendig sind um das entsprechende Gerät verwenden zu können, können nicht von allen Benutzern eingenommen werden. In ihrer Beweglichkeit eingeschränkte Personen, wie zum Beispiel ältere Menschen, Menschen mit Rückenbeschwerden oder schwangere Frauen, sind von dieser Problemstellung betroffen.

Eine Aufgabe der Erfindung liegt darin, ein Behandlungssystem mit einem Gerät bereitzustellen, durch das eine Fußpflege ermöglicht wird, ohne dass der Benutzer selbst das Gerät in einer Hand haltend zu dem zu (kosmetisch) behandelnden Fuß führen muss, noch ist eine zweite Person (Therapeut) notwendig, die ein handgehaltenes Gerät zu dem Fuß der zu behandelnden Person hinführt.

**Gelöst wird die Aufgabe** ein Behandlungssystem nach Anspruch 1.

Das Behandlungssystem kann zur kosmetischen Behandlung eines Fußes verwendet werden.

Das Behandlungssystem umfasst ein Behandlungsgerät und ein Bedienelement. Das Behandlungsgerät umfasst ein Basiselement und ein Schleifelement. Das Basiselement weist einen Motor und eine Schnittstelle des Bedienelements oder für das Bedienelement auf.

Das Schleifelement ist bewegbar auf dem Basiselement gelagert und kann von dem Motor angetrieben werden. Der Motor kann durch das Bedienelement gesteuert werden, wenn zwischen der Schnittstelle des Bedienelements und dem Bedienelement ein Abstand von mehr als 10 cm vorliegt.

Das Behandlungssystem ist geeignet einen Fuß eines Benutzers kosmetisch zu behandeln, beispielsweise kann durch das Behandlungssystem Hornhaut an dem Fuß des Benutzers reduziert oder entfernt werden, um den Fuß des Benutzers ästhetisch ansprechender zu machen.

Das Basiselement kann so ausgestaltet sein, dass dieses flächig auf einem Boden platziert werden kann und, dass sich das Schleifelement von dem Boden hinweg erstreckt. Beispielsweise kann das Behandlungsgerät auf Standfüßen auf dem Boden platziert sein, bevorzugt weist das Basiselement eine Standfläche zum Platzieren auf dem Boden auf.

Das Basiselement ist durch einen Menschen tragbar, insbesondere hat das Basiselement ein Gewicht von weniger als 10 kg. Speziell hat das Behandlungsgerät ein Gewicht von maximal 15 kg.

Der Motor des Basiselements kann ein Elektromotor sein.

Die Schnittstelle für das Bedienelement an dem oder in dem Basiselement kann eine Anschlussbuchse für einen Stecker sein. Die Schnittstelle kann auch einen Empfänger umfassen, durch den ein drahtloses Signal empfangen werden kann. Beispielsweise kann die Schnittstelle einen IR-Empfänger (Infrarot) umfassen.

Die Schnittstelle kann über eine Steuereinheit mit dem Motor verbunden sein.

Das Bedienelement kann ein Handbedienelement sein, das so ausgestaltet ist, dass es per Hand von einem Benutzer bedient werden kann. Durch das Bedienelement kann ein Signal an die Schnittstelle des Basiselements übertragen, insbesondere gesendet werden.

Das Bedienelement kann einen Sender umfassen, der ein drahtloses Signal erzeugt und aussendet. Das Signal kann von einem Empfänger der Schnittstelle empfangen werden.

Das Bedienelement kann über ein Kabel mit der Schnittstelle verbunden sein.

Das Schleifelement kann so auf dem Basiselement gelagert sein, dass es oszillationsfähig ist oder um eine Achse rotieren kann. Die Oszillation oder Rotation oder Drehung des Schleifelements (Bewegung) wird von dem Motor angetrieben.

Das Schleifelement besteht bevorzugt aus einem porösen Werkstoff. Der Werkstoff kann dabei aus einem synthetischen oder natürlichen Werkstoff geformt sein. Die Form des Schleifelements ist darauf ausgelegt den menschlichen Fuß aufnehmen zu können, insbesondere die Ferse und/oder den Ballen des Fußes.

Die Bewegung des Schleifelements kann durch das Bedienelement gesteuert werden. Dazu kann ein Benutzer ein Signal an dem Bedienelement verursachen, das an die Schnittstelle des Bedienelements an oder in dem Basiselement übertragen wird. Das Signal kann beispielsweise durch eine Steuereinheit oder eine Recheneinheit verarbeitet werden. Die Steuer- oder Recheneinheit erzeugt ein weiteres Signal, das an den Motor des Basiselements weitergeleitet wird und diesen steuert. Der Motor treibt auf Grundlage des Signals das Schleifelement an.

Das Bedienelement kann verschiedene Eingabeelemente umfassen. Beispielsweise kann das Bedienelement einen An-/Aus-Schalter umfassen. Das Bedienelement kann auch ein Eingabeelement zur Steuerung der Intensität der Bewegung des Schleifelements umfassen. Dabei kann die Oszillationsfrequenz oder die Rotationsgeschwindigkeit gesteuert werden.

Zwischen dem Bedienelement und der Schnittstelle des Bedienelements in oder an dem Basiselement kann ein Abstand von mehr als 10 cm vorhanden sein. Bevorzugt beträgt der Abstand mindestens 30 cm, noch bevorzugter beträgt der Abstand mindestens 50 cm.

Ist zwischen dem Bedienelement und der Schnittstelle ein Kabel angeordnet, kann das Kabel eine Länge von mindestens 10 cm, insbesondere 30 cm, speziell mindestens 50 cm, betragen. Das Bedienelement kann über dieses Kabel zwischen der Schnittstelle und dem Bedienelement mit elektrischem Strom versorgt werden.

Ist zwischen der Schnittstelle und dem Bedienelement kein (physisches) Kabel angeordnet, kann das Bedienelement einen Sender umfassen, der bevorzugt durch eine Stromversorgungseinheit (Akku) mit Energie versorgt wird. In diesem Fall umfasst die Schnittstelle einen Empfänger, der die von dem Sender des Bedienelements sendbaren Signale empfangen kann. Insbesondere kann zwischen dem Bedienelement und der Schnittstelle eine Daten- oder Signalübertragung auf Grundlage von IR-Signalen stattfinden.

Die Bewegung des Schleifelements kann stufenweise oder stufenlos durch das Bedienelement gesteuert werden.

Das Behandlungssystem ermöglicht einem Nutzer das Behandlungsgerät auf einer Fläche des Basiselements auf dem Boden abzustellen und auf einem Stuhl vor dem Behandlungsgerät sitzend das Behandlungsgerät über das Bedienelement zu steuern. Dabei kann der Nutzer einen zu behandelnden Fuß auf dem Schleifelement platzieren und über das Bedienelement die Bewegung des Schleifelements steuern, wodurch eine Behandlung des Fußes möglich ist, ohne das Schleifelement in einer Hand haltend zu dem Fuß zu führen und zu bewegen.

Das Schleifelement weist für zumindest einen Abschnitt einer Fläche des Schleifelements eine konkave Wölbung auf. Die konkave Wölbung ist insbesondere darauf ausgelegt, die Formen des menschlichen Fußes aufnehmen zu können.

Die zumindest abschnittsweise Fläche des Schleifelements, die eine konkave Wölbung aufweist, ist groß genug um mindestens einen Teil eines Fußes aufnehmen zu können, insbesondere groß genug für den Ballen, die Hacke und/oder den Fußaußenrand.

Die abschnittsweise Fläche mit konkaver Wölbung ist vorzugsweise so angeordnet, dass sie für einen sitzenden Benutzer gut zugänglich ist.

Durch die Größe, Form und Anordnung der konkaven Wölbung soll ein erleichtertes Abschleifen insbesondere konvexer Formen des menschlichen Fußes bewirkt werden.

Das Basiselement kann zumindest eine Abstellfläche für einen menschlichen Fuß aufweisen, bevorzugt zwei Abstellflächen für je einen menschlichen Fuß.

Die Abstellflächen sind vorzugsweise parallel zueinander und/oder in einer Ebene befindlich angeordnet.

Die Abstellflächen können beabstandet voneinander angeordnet sein, wobei der Abstand zwischen den Abstellflächen einen maximalen Abstand von 50 cm nicht überschreiten sollte, bevorzugt 15 cm, noch bevorzugter 8 cm beträgt.

Die Abstellflächen können gegenüber dem Boden eine Neigung aufweisen. Die Neigung sollte einen maximalen Winkel von 60 ° nicht überschreiten. Bevorzugt beträgt die Neigung einen Winkel von 40 °, noch bevorzugter einen Winkel von 25 °. Dabei sind die Abstellflächen vorzugsweise einem Benutzer entgegengeneigt.

Die Abstellflächen können eine Größe aufweisen, dass zumindest je ein Teil eines Fußes darauf abgestellt werden kann, speziell so groß, dass die gesamte Sohle je eines Fußes auf den Abstellflächen abgestellt werden kann.

Die zumindest eine Abstellfläche weist eine Fläche von mindestens 50 cm² auf, bevorzugt eine Fläche von 200 cm², noch bevorzugter eine Fläche von 250 cm².

Aus der Größe, der Neigung und der Anordnung der Abstellflächen ergibt sich ein verkürzter Weg von dem zu behandelnden Fuß hin zu dem Schleifelement, was die kosmetische Behandlung des Fußes erleichtert.

Die zumindest eine Abstellfläche weist eine Beschichtung mit einem Gleitreibungskoeffizienten µ von mindestens 0,3 auf. Der Gleitreibungskoeffizient µ ist nach der DIN 51131 zu bestimmen.

Die Beschichtung kann eine rutschfeste Farbe sein, ein Pulverlack und/oder eine Kunststoffbeschichtung.

Die Oberfläche der Beschichtung kann Noppen aufweisen, gelocht, aufgeraut oder gerillt sein.

Die Beschichtung bedeckt zumindest abschnittsweise die zumindest eine Abstellfläche, bevorzugt entspricht die Beschichtung der Größe und Form der zumindest einen Abstellfläche. Die Beschichtung soll das Abrutschen des Fußes verhindern.

Das Basiselement kann ein Beleuchtungselement aufweisen.

Das Beleuchtungselement kann so angeordnet werden, dass es für einen vor dem Behandlungsgerät sitzenden Nutzer sichtbar ist.

Das Beleuchtungselement kann eine Lampe und insbesondere eine Lampenabdeckung umfassen. Die Lampen-Abdeckung kann aus einem lichtdurchlässigen Werkstoff gefertigt sein, wie zum Beispiel Glas oder Kunststoff.

Die Lichtquelle des Beleuchtungselements bildet sich bevorzugt als Leuchtdiode (LED) aus. Das Beleuchtungselement kann sich als LED-Modul aus mehreren LEDs zusammensetzen.

Das Beleuchtungselement kann gegenüber dem Boden einen Neigungswinkel aufweisen. Der Neigungswinkel des Beleuchtungselements sollte nicht eine Neigung in Richtung des Bodens von 90 Grad überschreiten oder eine Neigung von dem Boden weg von 45 Grad überschreiten.

Die Lichtstärke des Beleuchtungselements kann mindestens 50 Lux betragen.

Das Basiselement mit zumindest einer Abstellfläche für einen menschlichen Fuß ist durch das Beleuchtungselement beleuchtbar.

Dabei kann zumindest ein Abschnitt der zumindest einen Abstellfläche von dem Beleuchtungselement beleuchtet werden. Speziell soll dabei mindestens das untere Drittel der zumindest einen Abstellfläche durch das Beleuchtungselement ausgeleuchtet werden.

Durch die Beleuchtung der zumindest einen Abstellfläche können die auf der zumindest einen Abstellfläche abgestellten Füße eines Benutzers durch den Benutzer begutachtet werden, um festzustellen, welche Stellen des Fußes im besonderen Maße von dem Schleifelement abgeschliffen werden sollten, um den Fuß ästhetisch ansprechender zu machen.

Das Behandlungssystem kann ein Nagelpflege-Handgerät aufweisen, das über ein Kabel mit dem Behandlungsgerät verbunden ist, insbesondere weist das Kabel eine Länge von mindestens (mehr als) 10 cm auf. Das Nagelpflege-Handgerät ist darauf ausgelegt mit einer Hand des Benutzers verwendet zu werden. Das Nagelpflege-Handgerät kann einen Ein- und einen Ausschalter umfassen.

Zum Einstellen der Drehgeschwindigkeit eines Nagelpflege-Handgerätaufsatzes kann das Nagelpflege-Handgerät einen Stufen- und/ oder Drehschalter aufweisen, um die Drehgeschwindigkeit in Stufen- oder stufenlos einstellen zu können.

Das Kabel, über das das Nagelpflege-Handgerät mit dem Behandlungsgerät verbunden ist, ist bevorzugt über eine Anschlussbuchse, die in das Basiselement integriert ist, verbunden. Die Länge des Kabels kann insbesondere so ausgelegt werden, dass ein vor dem Behandlungsgerät sitzender Benutzer das Nagelpflege-Handgerät in der Hand halten kann. Das Kabel des Nagelpflege-Handgeräts kann bevorzugt eine Länge von 40 cm, bevorzugter eine Länge von 50 cm aufweisen.

Ein Behandlungsgerät kann zur kosmetischen Behandlung eines Fußes verwendet werden. Insbesondere kann das Behandlungsgerät in ein Behandlungssystem integriert sein.

Ein Behandlungsgerät weist ein Basiselement, einen Motor und ein Schleifelement auf. Der Motor ist in das Basiselement integriert. Speziell kann es sich bei dem Motor um einen Elektromotor handeln. Das Schleifelement kann bewegbar auf dem Basiselement gelagert sein und von dem Motor angetrieben werden. Bei der Bewegung des Schleifelements kann es sich um eine oszillatorische und/oder rotatorische Bewegung handeln.

Das Schleifelement weist zumindest abschnittsweise eine Fläche auf, die eine konkave Wölbung aufweist.

Das Behandlungsgerät integriert in ein Behandlungssystem ist dazu geeignet, auf dem Fußboden abgestellt und auf einem Stuhl davor sitzenden Benutzer verwendet zu werden. Dabei erhöht die automatische Bewegbarkeit des Schleifelements den Bedienkomfort des Behandlungsgeräts. Die konkave Wölbung des Schleifelements erleichtert das Abschleifen konvexer Formen des menschlichen Fußes. Damit wird ebenfalls der Bedienkomfort des Behandlungsgeräts erhöht.

Das Schleifelement weist eine im Wesentlichen zylindrische Form auf, insbesondere kann das Schleifelement eine im Wesentlichen konische Form aufweisen, so dass die Fläche, die auf einem Trägerelement sitzt, einen größeren Durchmesser im Querschnitt betrachtet aufweist, als die Fläche, die die konkave Wölbung integriert.

Das Schleifelement weist einen Rand auf. Der Rand ist mindestens 5 mm breit. Der Rand kann eine zu der durch die Standfläche aufgespannten Ebene planparallele Form aufweisen oder bevorzugt konvex geformt sein. Ein konvex geformter Rand ist insbesondere dazu geeignet, die konkaven Formen eines menschlichen Fußes erreichen zu können. Der Rand ist von der Seitenfläche (Mantelfläche) des Schleifelements bis zu dem Punkt zu messen, ab dem die planparallele bzw. konvexe Fläche des Randes in die konkav gewölbte Fläche übergeht. Der Rand kann bevorzugter eine Breite von 8 mm, noch bevorzugter eine Breite von 12 mm aufweisen.

Die konkave Wölbung kann im Querschnitt betrachtet achsensymmetrisch bezogen auf eine Symmetrieachse sein.

Die Symmetrieachse kann sich aus einer gedachten Linie ergeben, die senkrecht auf einer Ebene steht, die durch die Erstreckung der Standfläche aufgespannt wird, und die den Scheitelpunkt der konkaven Wölbung schneidet.

Der Scheitelpunkt der konkaven Wölbung kann mindestens 5 mm von dem Rand in Richtung der Symmetrieachse beabstandet sein.

Die Tiefe der konkaven Wölbung, also der senkrechte Abstand zwischen dem Rand und dem Scheitelpunkt der konkaven Wölbung entlang der Symmetrieachse, beträgt bevorzugt 8 mm, noch bevorzugter 10 mm.

Die Konkavität des Schleifelements bezieht sich auf das Schleifelement selbst und nicht auf die Orientierung des Schleifelements.

Das Schleifelement ist in besonderer Weise durch die Achssymmetrie und die Tiefe der Wölbung dazu geeignet die im Wesentlichen konvexen Formen des menschlichen Fußes, wie beispielsweise den Ballen und die Hacke des Fußes, aufnehmen zu können.

Das Behandlungsgerät kann zur kosmetischen Behandlung eines Fußes verwendet werden. Insbesondere kann das Behandlungsgerät in ein Behandlungssystem integriert sein.

Ein Behandlungsgerät weist ein Basiselement, einen Motor und ein Schleifelement auf. Der Motor ist in das Basiselement integriert. Speziell kann es sich bei dem Motor um einen Elektromotor handeln. Das Schleifelement kann bewegbar auf dem Basiselement gelagert sein und von dem Motor angetrieben werden.

Das Basiselement weist eine Standfläche zum flächigen Platzieren auf einer ebenen Fläche auf. Die Standfläche weist eine Fläche von mindestens 100 cm², bevorzugt 500 cm², noch bevorzugter 900 cm² auf.

Das Basiselement weist ein Gewicht von mindestens 0,5 kg, bevorzugt 1,5, noch bevorzugter von 2,0 kg auf.

Durch das Gewicht des Basiselements kann insbesondere die Gleitreibung zwischen dem Fußboden und der Standfläche des Behandlungsgeräts eingestellt werden. Insbesondere höhere Gewichte (bspw. 1,5, 2,0 oder größer) bewirken eine Vergrößerung der Normalkraft, die von der Standfläche des Behandlungsgerät auf den Fußboden wirkt, und erhöhen damit die Gleitreibung. Eine erhöhte Gleitreibung erschwert eine unerwünschte Verschiebung des Behandlungsgeräts bei einer Behandlung.

Die Standfläche kann zumindest teilweise mit einer Beschichtung versehen sein.

Die Beschichtung kann insbesondere eine hohe Oberflächenrauigkeit aufweisen. Eine erhöhte Oberflächenrauigkeit der Beschichtung bewirkt eine Erhöhung der Gleitreibung zwischen Behandlungsgerät und Boden und kann dazu beitragen eine Verschiebung des Behandlungsgeräts zu verhindern oder zumindest zu erschweren.

Die Erstreckung der Standfläche in einer durch die Standfläche aufgespannten Ebene kann größer sein als die Erstreckung des Behandlungsgeräts senkrecht zu der Ebene.

Die Größe der Standfläche in Kombination damit, dass das Behandlungsgerät im Wesentlichen breiter als hoch ist (größere Erstreckung der Standfläche als die Erstreckung des Behandlungsgeräts senkrecht zu der Standfläche) verschiebt den Schwerpunkt des Geräts so, dass eine Verkippung oder ein Umkippen des Behandlungsgeräts verhindert oder zumindest erschwert wird.

Das Schleifelement kann lösbar mit dem Basiselement verbunden sein. Insbesondere kann das Schleifelement formschlüssig mit dem Basiselement verbunden sein.

Das Schleifelement kann bei einer Behandlung durch zumindest zwei Sicherungen gegen ein Abheben entlang der Symmetrieachse von dem Basiselement weg gesichert werden.

Bei den zumindest zwei Sicherungen kann es sich um Sicherungen handeln, die durch form- und/oder kraftschlüssige Elemente realisiert werden. Beispielsweise kann ein Abheben des Schleifelements (bzw. des Schleifelementaufsatzes) durch einen mechanischen Mechanismus verhindert werden, bei dem Haken in oder an dem Schleifelement oder Schleifelementsaufsatz ein- oder angreifen.

Die zumindest zwei Sicherungen können über einen Schalter zum Steuern der Sicherungen gesteuert werden. Der Schalter zum Steuern der Sicherungen kann das Lösen und das An- bzw. Eingreifen der Sicherungen an dem Schleifelement bzw. an dem Schleifelementaufsatz steuern. Bei bspw. durch mechanisch realisierte Sicherungen als an dem Schleifelement eingreifenden Haken, kann der Schalter zum Steuern der Sicherungen bei Betätigung das Lösen der Haken von dem Schleifelement bewirken, so dass das Schleifelement integriert in einem Schleifelementaufsatz aus der Lagerung im Basiselement entfernt werden kann.

Die zumindest zwei Sicherungen können bevorzugt so ausgestaltet sein, dass die Verbindung zwischen Schleifelement bzw. Schleifelementaufsatz und dem Basiselement lösbar ist, insbesondere nur dann lösbar ist, wenn das Schleifelement nicht von dem Motor angetrieben wird, um Verletzungen eines Benutzers zu vermeiden.

Das Schleifelement kann eine zumindest abschnittsweise zylinderförmige Außenfläche aufweisen. Die Außenfläche kann einen Durchmesser von zumindest 20,0 mm, bevorzugt zumindest 40,0 mm, besonders bevorzugt zumindest 60,0 mm, bevorzugter zumindest 80,0 mm, am meisten bevorzugt zumindest 100,0 mm, aufweisen.

Das Behandlungsgerät kann zur kosmetischen Behandlung eines Fußes verwendet werden. Insbesondere kann das Behandlungsgerät in ein Behandlungssystem integriert sein.

Jedes der hierin offenbarten Behandlungsgeräte oder Behandlungssysteme kann zur kosmetischen Behandlung eines (menschlichen) Fußes werden.

Die Ausführungsformen der Erfindung sind anhand von einem Beispiel dargestellt jedoch nicht auf eine Weise, in der Beschränkungen aus den Figuren in die Patentansprüche übertragen oder hineingelesen werden. Gleiche Bezugszeichen in den Figuren geben gleiche Elemente an.
- Figur 1: zeigt ein Behandlungssystem 100, dargestellt in einer isometrischen Perspektive;
- Figur 2: zeigt ein Behandlungsgerät 200 in einer schematischen Draufsicht;
- Figur 3: zeigt das Behandlungsgerät 200 in einer schematischen Vorderansicht;
- Figur 4: zeigt das Behandlungsgerät 200 in einer schematischen Seitenansicht;
- Figur 5: zeigt das Behandlungsgerät 200 in einer schematischen Rückansicht;
- Figur 6: zeigt einen Schleifelementaufsatz 400 in einer geschnittenen Ansicht;
- Figur 7: zeigt den Schleifelementaufsatz 400 in Eingriff mit einem Basiselement 300 in einem Teilschnitt.

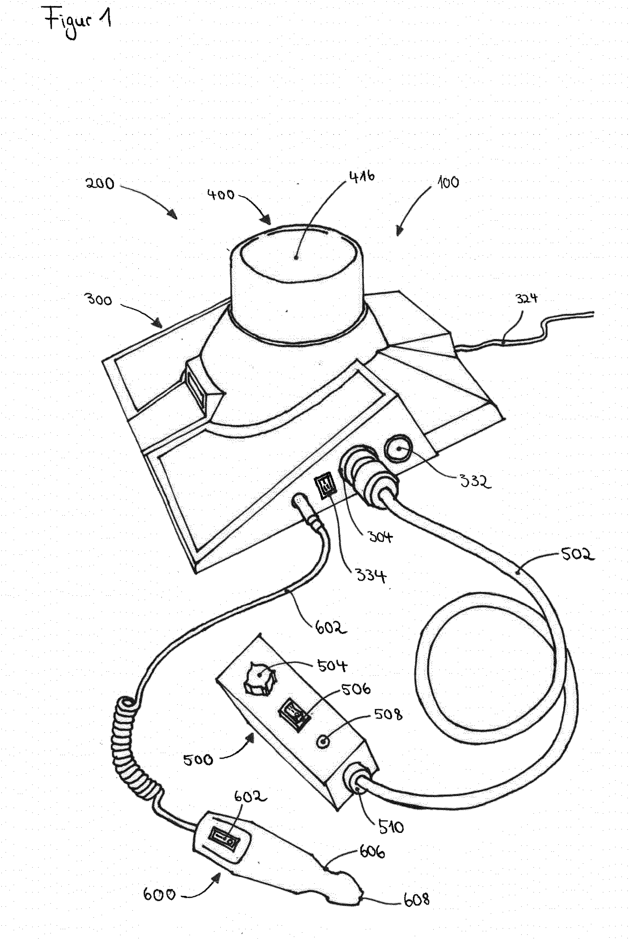

Figur 1 zeigt ein Behandlungssystem 100. Das Behandlungssystem 100 umfasst ein Behandlungsgerät 200, ein Bedienelement 500 und ein Nagelpflege- Handgerät 600.

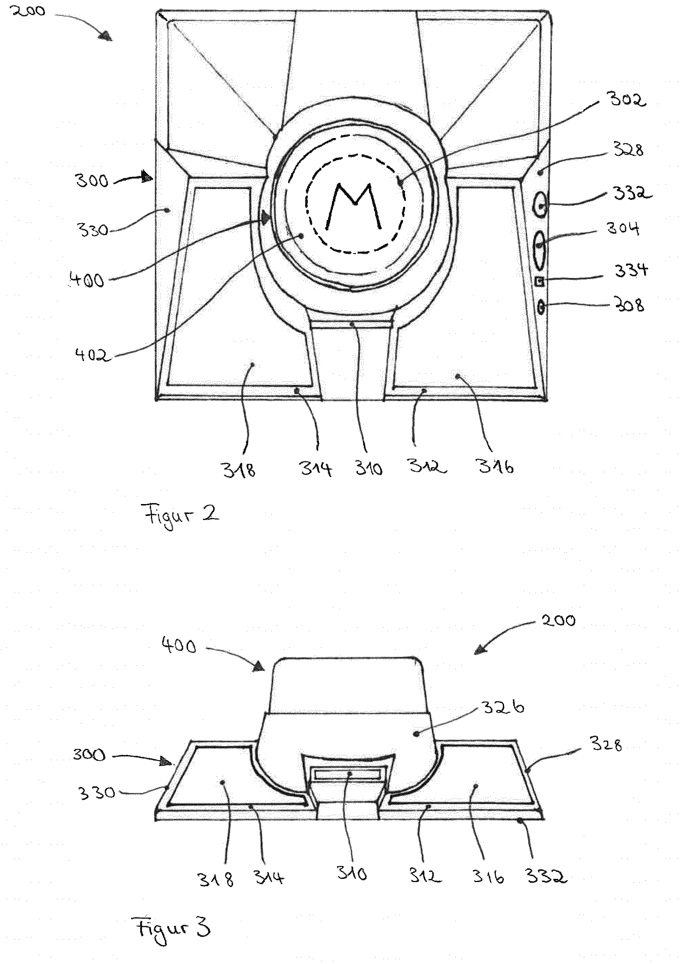

Das Behandlungsgerät 200 umfasst ein Basiselement 300 und ein Schleifelementaufsatz 400 mit Schleifelement 402. Das Behandlungsgerät 200 ist über ein Netzkabel 324 mit einer Stromversorgung verbunden. Das Behandlungsgerät 200 wird mit Blick auf die Figuren 2 bis 6 ausführlich beschrieben.

Das Bedienelement 500 umfasst einen Kabelanschluss 510, an dem ein Kabel 502 angeschlossen, wobei durch das Kabel 502 das Bedienelement 500 mit dem Behandlungsgerät 200, insbesondere mit dem Basiselement 300, so verbunden ist, dass Signale zwischen dem Bedienelement 500 und dem Behandlungsgerät 200 ausgetauscht werden können. Über das Kabel 502 kann das Bedienelement 500 auch mit Strom versorgt werden.

Das Bedienelement 500 umfasst einen Drehschalter 504. Durch den Drehschalter 504 kann die Bewegung des Schleifelements 402 oder die Bewegung eines Aufsatzes (nicht abgebildet), der im Nagelpflege-Handgerät 600 eingesetzt werden kann, gesteuert werden. Insbesondere kann durch den Drehschalter 504 die Bewegung des Schleifelements 402 oder des Aufsatzes im Nagelpflege-Handgerät 600 stufenlos gesteuert werden. Dies wird mit Blick auf Figur 2 ausführlicher beschrieben.

Das Bedienelement 500 umfasst weiterhin einen Kippschalter 506. Durch den Kippschalter 506 kann die Bewegung des Schleifelements 402 oder die Bewegung eines Aufsatzes im Nagel-Pflege-Handgerät 600 an- oder ausgestellt werden, insbesondere kann durch den Kippschalter 506 die Drehrichtung (bidirektional) eingestellt werden.

Das Bedienelement 500 umfasst ein Anzeigeelement 508.Das Anzeigeelement 508 kann eine LED sein. Das Anzeigeelement 508 kann so konfiguriert sein, dass es mit höher eingestellter Bewegungsintensität (mittels Drehschalter 504) des Schleifelements 402, bzw. des Aufsatzes im Nagel-pflege-Handgeräts 600, ebenfalls mit höherer Intensität leuchtet.

Das Nagelpflege-Handgerät 600 umfasst einen Kabelanschluss, an dem ein Kabel 602 angeschlossen ist. Das Kabel 602 verbindet das Nagelpflege-Handgerät 600 mit Behandlungsgerät 200, insbesondere mit dem Basiselement 300 des Behandlungsgeräts 200. Über das Kabel 602 kann das Nagelpflege-Handgerät 600 mit Strom versorgt werden.

Das Nagelpflege-Handgerät 600 umfasst einen Kippschalter 602, durch den das Nagelpflege-Handgerät 600 eingeschaltet oder ausgeschaltet werden kann.

Das Nagelpflege-Handgerät 600 umfasst weiterhin eine Aufsatzaufnahme 608. Die Aufsatzaufnahme 608 kann an einem Ende des Nagelpflege-Handgeräts 600 angeordnet sein. In die Aufsatzaufnahme 608 können verschiedene Aufsätze zur Nagelpflege eingesetzt werden. Solche Aufsätze können beispielsweise sein, ein Sandpapieraufsatz, Saphirkegeln, Zylinderfräser, Flammenfräser, Filzkegel, Nadelfräser, oder eine Reinigungsbörse.

Das Nagelpflege-Handgerät 600 kann weiterhin einen Drehschalter (nicht in Figur 1 dargestellt) umfassen. Durch den Drehschalter kann (alternativ zu Drehschalter 504) die Bewegungsintensität eines Aufsatzes in der Aufsatzaufnahme 608 gesteuert werden. Insbesondere kann durch den Drehschalter die Bewegungsintensität eines Aufsatzes in der Aufsatzaufnahme 608 stufenlos gesteuert werden.

Das Nagelpflege-Handgerät 600 umfasst eine Griffmulde 606- Die Griffmulde 606 ist bevorzugt in der Nähe (Abstand weniger als 10 cm) der Aufsatzaufnahme 608 angeordnet. Durch die Griffmulde 606 kann ein Benutzer das Nagelpflege-Handgerät 600 sicher halten.

Figur 2 zeigt das Behandlungsgerät 200, bestehend aus dem Basiselement 300 und dem Schleifelementaufsatz 400. Das Basiselement 300 weist einen Motor 302, eine Schnittstelle 304 für ein Bedienelement 500, einen Schalter 332, einen Kippschalter 334 und einen Anschluss für ein Peripheriegerät 308, insbesondere einen Anschluss für ein Nagelpflege-Handgerät 600, auf. Darüber hinaus umfasst das Basiselement 300 Abstellflächen 312 und 314 sowie Beschichtungen 316 und 318 und ein Beleuchtungselement 310, auf das in der Figurenbeschreibung von Figur 3 tiefer eingegangen werden soll.

Über die Schnittstelle 304 kann das Bedienelement 500 mit dem Basiselement 300 verbunden werden, über das der Motor 302 gesteuert werden kann. Durch den Motor 302 wird die Bewegungsintensität des Schleifelementaufsatzes 400 bzw. eines darauf angebrachten Schleifelements 402 eingestellt.

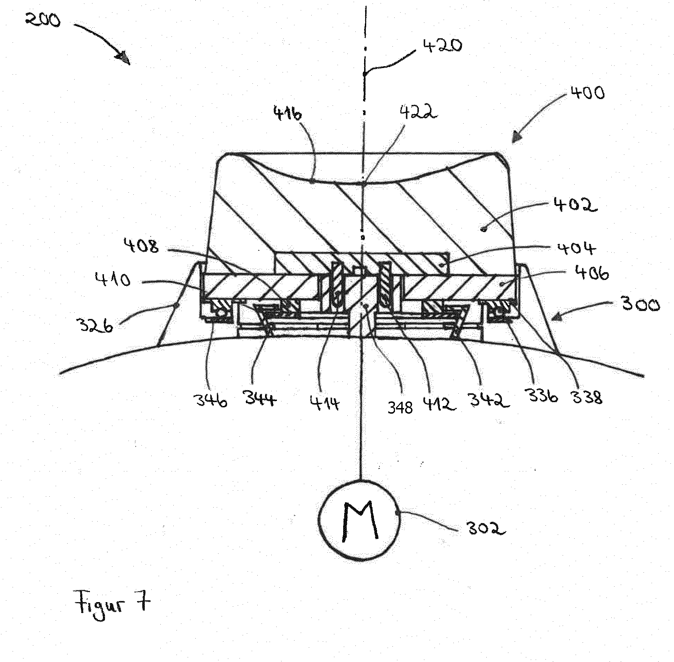

Über Kippschalter 334 kann zwischen verschiedenen Betriebsmodi des Behandlungsgeräts 200 geschalten werden, insbesondere zwischen einem Betriebsmodus in dem das Schleifelement 402 bewegbar ist und einem Betriebsmodus in dem ein Aufsatz in dem Nagelpflege-Handgerät 600 bewegbar ist. Die Funktionsweise von Schalter 332 wird in Hinblick auf Figur 7 erläutert.

Weiterhin umfasst das Basiselement 300 eine konusförmige Lagerung 326, in der der Schleifelementaufsatz 400 gelagert ist.

Der Schleifelementaufsatz 400 weist von oben betrachtet eine im Wesentlichen kreisförmige Form auf.

Die Abstellflächen 312 und 314 weisen eine im Wesentlichen rechteckige Form auf, die von der konusförmigen Lagerung 326 geschnitten werden. Auf den Abstellflächen 312 und 314 sind Beschichtungen 316 und 318 angebracht.

Die Beschichtungen 316 und 318 sind in ihrer Form und Größe im Wesentlichen an die Abstellflächen 312 und 314 angepasst, was bedeutet, dass die Beschichtungen 316 und 318 zumindest einen Teil der Abstellflächen 312 und 314 bedeckt, bevorzugt mindestens 50 % der Abstellflächen 312 und 314 bedeckt.

Figur 3 zeigt das Behandlungsgerät 200 in einer Frontalansicht. Das Beleuchtungselement 310 des Basiselements 300, integriert in das Behandlungsgerät 200, ist in der konusförmigen Lagerung 326 angeordnet.

Speziell ist das Beleuchtungselement 310 im unteren Drittel der konusförmigen Lagerung 326 herausstehend angeordnet. Das Beleuchtungselement 310 ist so angeordnet, dass mindestens das untere Drittel der Abstellflächen 312 und 314 bzw. das untere Drittel der Beschichtungen 316 und 318 beleuchtet werden.

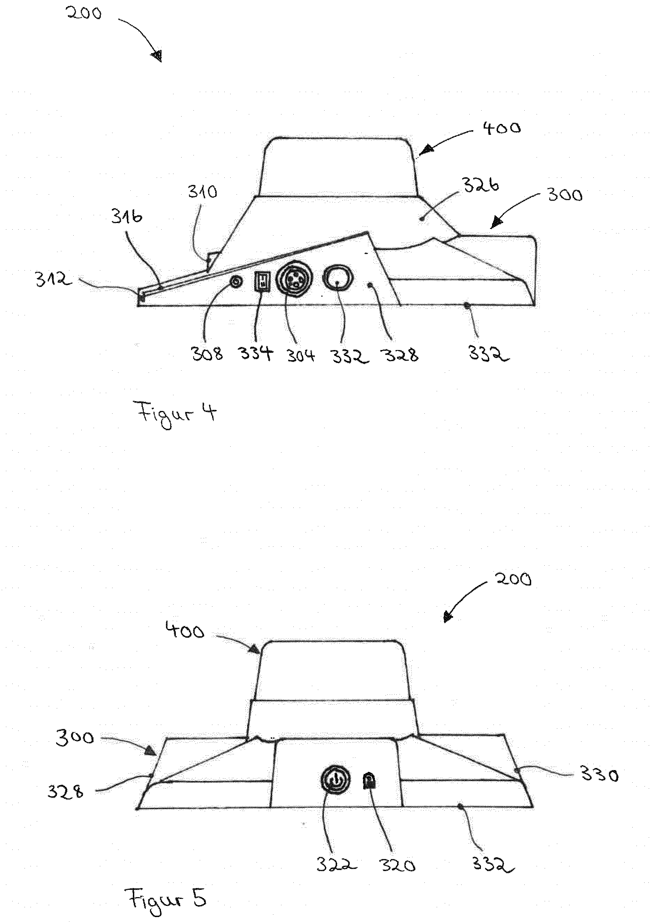

Die Abstellflächen 312 und 314 befinden sich in einer Ebene liegend und können einen Neigungswinkel gegenüber der Standfläche des Basiselements 300 aufweisen. Dieser Zusammenhang wird in Figur 4 verdeutlicht.

Das Basiselement 300 weist eine Standfläche 332 auf.

Figur 4 zeigt das Behandlungsgerät 200 in einer Seitenansicht. In der Seitenansicht des Behandlungsgeräts 200 wird die Neigung der Abstellflächen 312 und 314, insbesondere in dieser Darstellung der Abstellfläche 312, deutlich. Die Neigung der Abstellflächen 312 und 314 gegenüber der Standfläche des Basiselements 300 beträgt bevorzugt einen Winkel zwischen 5 ° und 80°, noch bevorzugter einen Winkel von 25 °.

Der obere Rand der konusförmigen Lagerung 326 weist einen durchschnittlich kleineren Radius auf, als der untere Rand der konusförmigen Lagerung, wobei der Steigungswinkel, betrachtet zwischen der Standfläche 332 des Basiselements 300 und der Außenfläche der konusförmigen Lagerung 326, kleiner ist als in der Vorderansicht, wodurch sich von oben betrachtet eine im Wesentlichen elliptische Form der konusförmigen Lagerung 326 ergibt.

Die Schnittstelle 304 für ein Bedienelement ist in einer Seitenfläche 328 oder 330 angeordnet (nach Figur 2 - 5 in Seitenfläche 328), die sich aus dem Neigungswinkel der Abstellflächen 312 und 314 gegenüber der Standfläche des Basiselements 300 ergibt.

Dabei ist die Schnittstelle in oder an dem Basiselement 300 integriert. Der Anschluss für ein Peripheriegerät 308, bevorzugt ein Nagelpflege-Handgerät 600, ist ebenfalls in einer der Seitenflächen 328 oder 330 des Basiselements 300 angeordnet.

Figur 5 zeigt das Behandlungsgerät 200 aus einer Rückansicht. Das Basiselement 300 des Behandlungsgeräts 200 weist einen Anschluss 320 für den Stecker eines Netzadapterkabels 324 und einen Ein- und Ausschalter 322 auf. Der Ein-/Ausschalter 322 kann ein Beleuchtungselement integrieren, um anzuzeigen ob das Behandlungsgerät 200 betriebsfähig ist.

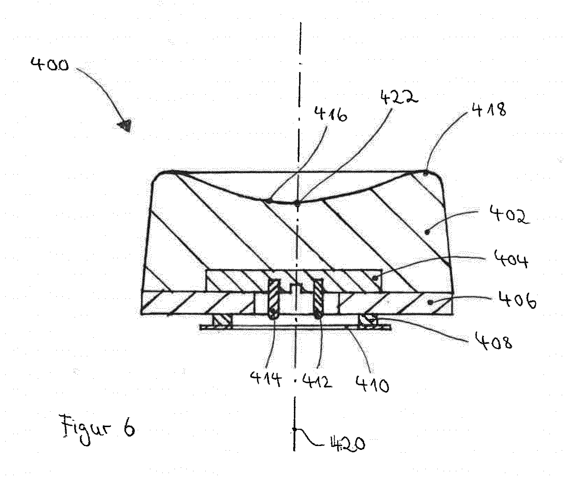

Figur 6 zeigt den Schleifelementaufsatz 400 in einer geschnittenen Ansicht. Der Schleifelementaufsatz 400 umfasst das Schleifelement 402, ein Stiftträgerelement 404, ein Trägerelement 406, einen Abstandhalter 408, einen Aufsatzring 410 und Stifte 412 und 414. Der Querschnitt des Schleifelementaufsatzes 400 bezieht sich auf eine Symmetrieachse 420.

Der abgebildete Schleifelementaufsatz 400 ist für eine Rotation um die Symmetrieachse 420 ausgelegt.

Das Schleifelement 402 weist nutzerseitig eine konkave Wölbung 416 und einen Rand 418 auf. Die konkave Wölbung umfasst einen Scheitelpunkt 422. Die konkave Wölbung 416 ist achssymmetrisch zu der Symmetrieachse 420. Der Scheitelpunkt 422 der konkaven Wölbung 416 ist mindestens um 5 mm gegenüber dem Rand 418 in Richtung der Symmetrieachse 420 verschoben, bevorzugt 8 mm, noch bevorzugter 10 mm.

Der Rand 418 kann eine konvexe Wölbung aufweisen. Insbesondere hat der Rand 418 eine Breite von mindestens 5 mm, bevorzugt eine Breite von 8 mm, noch bevorzugter eine Breite von 10 mm.

Das Schleifelement 402 kann am oberen Rand 418 einen kleineren Radius aufweisen als am unteren Rand des Schleifelements 402, wodurch das Schleifelement 402 im Wesentlichen durch eine konische Form geprägt ist.

Der kleinere Radius kann 10,0 mm betragen, bevorzugt zumindest 20,0 mm, besonders bevorzugt zumindest 50,0 mm. Der größere Radius kann zumindest 5,0 % größer sein als der kleinere Radius.

Das Schleifelement kann eine zylinderförmige oder zumindest abschnittsweise zylinderförmige Außenfläche aufweisen. Ein Durchmesser der Außenfläche kann zumindest 20,0 mm, bevorzugt zumindest 40,0 mm, besonders bevorzugt zumindest 60,0 mm, bevorzugter zumindest 80,0 mm, am meisten bevorzugt zumindest 100,0 mm, betragen.

Das Schleifelement 402 kann flächig auf dem Stiftträgerelement 404 und dem Trägerelement 406 angebracht sein, wobei das Stiftträgerelement 404 und das Trägerelement 406 kraft-, stoff- und/ oder formschlüssig miteinander verbunden sind.

Die Stifte 412 und 414 können ebenfalls per Kraft-, Stoff- und/ oder Formschluss mit dem Stiftträgerelement 404 verbunden sein.

Stiftträgerelement 404, Trägerelement 406, Abstandhalter 408, Aufsatzring 410 und die Stifte 412 und 414 sind dazu ausgelegt, in der konusförmigen Lagerung 326 des Basiselements 300 gelagert werden zu können, wobei das Schleifelement 402 senkrecht zur Standfläche 332 des Basiselements 300 betrachtet aus der konusförmigen Lagerung 326 herausragt.

Figur 7 zeigt den Schleifelementaufsatz 400 in Eingriff mit dem Basiselement 300 in einem Teilschnitt des Behandlungsgeräts 200. Die konusförmige Lagerung 326 des Basiselements 300 umfasst ein Kugellager 336, eine Auflagerung 348, über die das Antriebsmoment des Motors 302 auf den Schleifelementaufsatz 400 übertragen wird, und zwei Sicherungen 342, 344.

Das Kugellager 336 umfasst einen Ring 346, der in der konusförmigen Lagerung 326 integriert ist und einen Ring 338, der in Kontakt mit dem Trägerelement 406 des Schleifelementaufsatzes 400 steht. Das Kugellager 336 ist insbesondere dazu geeignet den Schleifelementaufsatz 400 in Kombination mit der Auflagerung 348 zu stützen, bevorzugt den Schleifelementaufsatz 400 gegen Verkippung bezüglich der Symmetrieachse 420 zu sichern.

Über die Auflagerung 348 wird das Antriebsmoment des Motors 302 auf den Schleifelementaufsatz 400 übertragen. Die Auflagerung 348 ist so ausgelegt, dass der Schleifelementaufsatz 400 drehfest bezüglich der Auflagerung 348 ist. In der abgebildeten Ausführungsform weist die Auflagerung 348 dafür Aussparungen auf, die in Eingriff mit den Stiften 412,414 stehen und den Schleifelementaufsatz 400 gegen Verdrehung gegenüber der Auflagerung 348 sichern.

Alternativ kann der Schleifelementaufsatz 400 über einen Dreh- und/oder Schnappverschluss, wie bspw. ein Bajonettverschluss, in Eingriff mi der Auflagerung 348 des Basiselements 300 gebracht werden.

Die zwei Sicherungen 342, 344 stehen in Eingriff mit dem Aufsatzring 410 des Schleifelementaufsatzes 400 und sichern den Schleifelementaufsatz 400 gegen Verschiebungen in Richtung der Symmetrieachse 420 weg von dem Basiselement 300. Die Sicherungen 342, 344 können mit dem Schalter 332 gesteuert werden.

Insbesondere kann es sich bei dem Schalter 332 zum Steuern der Sicherungen um einen Druckschalter handeln. Der Druckschalter kann so konfiguriert sein, dass bei Betätigung der Ein- oder Angriff der Sicherungen 342, 344 von dem Schleifelementaufsatz 400 gelöst wird. Bspw. würden bei Betätigung des Schalters 332 sich die in Figur 7 dargestellten Sicherungen 342, 344 radial nach außen von der Symmetrieachse 420 weg bewegen, so dass die Sicherungen 342, 344 nicht mehr in Eingriff mit dem Aufsatzring 410 des Schleifelementaufsatz 400 befinden und der Schleifelementaufsatz 400 aus der konusförmigen Lagerung 326 entnommen werden kann.

## Patentansprüche

1. **Behandlungssystem** mit einem Behandlungsgerät (200) zur Behandlung eines Fußes und einem Bedienelement (500), das Behandlungsgerät (200) mit:
(a) einem Basiselement (300);
(b) einem Motor (302), wobei der Motor (302) in das Basiselement (300) integriert ist;
(c) einem Schleifelement (402), wobei das Schleifelement (402) bewegbar auf dem Basiselement (300) gelagert ist und von dem Motor (302) antreibbar ist;
(d) wobei das Basiselement (300) eine Standfläche (332) zum flächigen Platzieren auf einer ebenen Fläche aufweist, wobei die Standfläche (332) eine Fläche von mindestens 100 cm² aufweist, und eine Schnittstelle (304) für das zugehörige Bedienelement (500);
**dadurch gekennzeichnet, dass** der Motor (302) von dem Bedienelement (500) auch dann steuerfähig ist, wenn ein Abstand zwischen der Schnittstelle (304) und dem Bedienelement (500) mehr als 10 cm beträgt.

2. Behandlungssystem nach Anspruch 1, wobei das Basiselement (300) ein Gewicht von mindestens 0,5 kg, bevorzugt 1,5 kg, noch bevorzugter 2,0 kg aufweist.

3. Behandlungssystem nach einem der vorangegangenen Ansprüche, wobei die Erstreckung der Standfläche (332) in einer durch die Standfläche (332) aufgespannten Ebene größer ist als die Erstreckung des Behandlungsgeräts senkrecht zu der Ebene.

4. Behandlungssystem nach einem der vorangegangenen Ansprüche, wobei das Schleifelement (402) durch zumindest zwei Sicherungen (342,344) gegen ein Abheben entlang der Symmetrieachse (420) von dem Basiselement (300) weg gesichert ist, wobei die zumindest zwei Sicherungen (342,344) über einen Schalter (332) zum Steuern der Sicherungen steuerbar sind.

5. Behandlungssystem nach einem der vorangegangenen Ansprüche, wobei die Standfläche (332) eine Fläche von 500 cm², bevorzugt von 900 cm², aufweist.

6. Behandlungssystem nach einem der vorangegangenen Ansprüche, wobei zumindest ein Abschnitt einer Fläche des Schleifelements (402) eine konkave Wölbung (416) aufweist.

7. Behandlungssystem nach einem der vorangegangenen Ansprüche, wobei das Schleifelement (402) einen Rand (418) aufweist, wobei der Rand (418) mindestens 5 mm breit ist.

8. Behandlungssystem nach einem der vorangegangenen Ansprüche, wobei die konkave Wölbung (416) im Querschnitt betrachtet achssymmetrisch bezogen auf eine Symmetrieachse (420) ist und der Scheitelpunkt (422) der konkaven Wölbung (416) mindestens 5 mm von dem Rand in Richtung der Symmetrieachse (420) beabstandet ist.

9. Behandlungssystem nach einem der vorangegangenen Ansprüche, wobei das Basiselement (300) zumindest eine Abstellfläche für einen menschlichen Fuß (312,314) aufweist, bevorzugt zwei Abstellflächen für je einen menschlichen Fuß (312,314).

10. Behandlungssystem nach Anspruch 9, wobei die zumindest eine Abstellfläche (312,314) eine Fläche von mindestens 50 cm² aufweist oder beide der Abstellflächen (312,314) je eine Fläche von mindestens 50 cm² aufweisen.

11. Behandlungssystem nach Anspruch 9 oder 10, wobei die zumindest eine Abstellfläche (312,314) eine Beschichtung (316,318) aufweist, so dass diese einen Gleitreibungskoeffizienten µ von mindestens 0,30 aufweist, bestimmt nach DIN 51131.

12. Behandlungssystem nach einem der vorangegangenen Ansprüche, wobei das Basiselement (300) ein Beleuchtungselement (310) aufweist, bevorzugt ist die zumindest eine Abstellfläche für den menschlichen Fuß (312,314) des Basiselements (300) durch das Beleuchtungselement (310) beleuchtbar.

13. Behandlungssystem nach einem der vorangegangenen Ansprüche, wobei das Behandlungssystem (100) ein Nagelpflege-Handgerät (600) aufweist, das über ein Kabel (602) mit dem Behandlungsgerät (200) verbunden ist, insbesondere das Kabel (602) eine Länge von mindestens 10 cm aufweist.

14. Behandlungssystem nach einem der vorangegangenen Ansprüche, wobei das Schleifelement (402) eine zumindest abschnittsweise zylinderförmige Außenfläche aufweist und die Außenfläche einen Durchmesser von zumindest 20,0 mm, bevorzugt zumindest 40,0 mm, besonders bevorzugt zumindest 60,0 mm, bevorzugter zumindest 80,0 mm, am meisten bevorzugt zumindest 100,0 mm, aufweist.

## Claims

1. **Treatment system** comprising a treatment device (200) for treating a foot and a control element (500), the treatment device (200) having
(a) a base element (300);
(b) a motor (302), wherein the motor (302) is integrated into the base member (300);
(c) a loop element (402), wherein the loop element (402) is movably mounted on the base element (300) and is drivable by the motor (302);
(d) wherein the base member (300) has a footprint (332) for flat placement on a flat surface, the footprint (332) having a surface area of at least 100 cm² , and an interface (304) for the associated control element (500);
**characterized in that** the motor (302) is controllable by the control element (500) even if a distance between the interface (304) and the control element (500) is more than 10 cm.

2. The treatment system according to claim 1, wherein the base element (300) has a weight of at least 0.5 kg, preferably 1.5 kg, more preferably 2.0 kg.

3. Treatment system according to any one of the preceding claims, wherein the extension of the footprint (332) in a plane spanned by the footprint (332) is greater than the extension of the treatment device perpendicular to said plane.

4. Treatment system according to any one of the preceding claims, wherein the loop element (402) is secured against lifting along the axis of symmetry (420) away from the base element (300) by at least two fuses (342,344), wherein the at least two fuses (342,344) are controllable via a switch (332) for controlling the fuses.

5. Treatment system according to any of the preceding claims, wherein the standing surface (332) has an area of 500 cm², preferably 900 cm².

6. Treatment system according to any one of the preceding claims, wherein at least a portion of a surface of the abrasive element (402) has a concave curvature (416).

7. A treatment system according to any one of the preceding claims, wherein the loop member (402) has a rim (418), the rim (418) being at least 5 mm wide.

8. A treatment system according to any one of the preceding claims, wherein the concave curvature (416) is axisymmetric with respect to an axis of symmetry (420) when viewed in cross-section and the apex (422) of the concave curvature (416) is spaced at least 5 mm from the edge in the direction of the axis of symmetry (420).

9. Treatment system according to any one of the preceding claims, wherein the base element (300) has at least one support surface for a human foot (312, 314), preferably two support surfaces for one human foot (312, 314) each.

10. The treatment system of claim 9, wherein the at least one storage surface (312,314) has an area of at least 50 cm² or both of the storage surfaces (312,314) each have an area of at least 50 cm².

11. Treatment system according to claim 9 or 10, wherein the at least one depositing surface (312, 314) has a coating (316, 318) such that it has a coefficient of sliding friction µ of at least 0.30, determined according to DIN 51131.

12. Treatment system according to any one of the preceding claims, wherein the base element (300) comprises an illumination element (310), preferably the at least one human foot resting surface (312, 314) of the base element (300) is illuminatable by the illumination element (310).

13. Treatment system according to any one of the preceding claims, wherein the treatment system (100) comprises a nail care hand tool (600) connected to the treatment device (200) via a cable (602), in particular the cable (602) has a length of at least 10 cm.

14. Treatment system according to any one of the preceding claims, wherein the loop element (402) has an at least partially cylindrical outer surface and the outer surface has a diameter of at least 20.0 mm, preferably at least 40.0 mm, more preferably at least 60.0 mm, more preferably at least 80.0 mm, most preferably at least 100.0 mm.

## Revendications

1. **Système de traitement** avec un appareil de traitement (200) pour le traitement d'un pied et un élément de commande (500), l'appareil de traitement (200) avec
(a) un élément de base (300) ;
(b) un moteur (302), le moteur (302) étant intégré dans l'élément de base (300) ;
(c) un élément de boucle (402), l'élément de boucle (402) étant monté de manière mobile sur l'élément de base (300) et pouvant être entraîné par le moteur (302) ;
(d) l'élément de base (300) présentant une surface d'appui (332) destinée à être placée à plat sur une surface plane, la surface d'appui (332) présentant une surface d'au moins 100 cm² , et une interface (304) pour l'élément de commande (500) associé ;
**caractérisé en ce que** le moteur (302) peut être commandé par l'élément de commande (500) même si une distance entre l'interface (304) et l'élément de commande (500) est supérieure à 10 cm.

2. Système de traitement selon la revendication 1, dans lequel l'élément de base (300) a un poids d'au moins 0,5 kg, de préférence 1,5 kg, encore plus préférablement 2,0kg.

3. Système de traitement selon l'une quelconque des revendications précédentes, dans lequel l'étendue de la surface d'appui (332) dans un plan délimité par la surface d'appui (332) est supérieure à l'étendue de l'appareil de traitement perpendiculairement audit plan.

4. Système de traitement selon l'une quelconque des revendications précédentes, dans lequel l'élément de boucle (402) est protégé par au moins deux fusibles (342, 344) contre un soulèvement le long de l'axe de symétrie (420) à l'écart de l'élément de base (300), lesdits au moins deux fusibles (342, 344) pouvant être commandés par un commutateur (332) pour commander les fusibles.

5. Système de traitement selon l'une des revendications précédentes, dans lequel la surface d'appui (332) présente une surface de 500 cm², de préférence de 900 cm².

6. Système de traitement selon l'une quelconque des revendications précédentes, dans lequel au moins une partie d'une surface de l'élément abrasif (402) présente une courbure concave (416).

7. Système de traitement selon l'une quelconque des revendications précédentes, dans lequel l'élément abrasif (402) comprend un bord (418), ledit bord (418) ayant une largeur d'au moins 5 mm.

8. Système de traitement selon l'une quelconque des revendications précédentes, dans lequel la voûte concave (416), vue en coupe transversale, est axisymétrique par rapport à un axe de symétrie (420), et le sommet (422) de la voûte concave (416) est distant d'au moins 5 mm du bord dans la direction de l'axe de symétrie (420).

9. Système de traitement selon l'une des revendications précédentes, dans lequel l'élément de base (300) présente au moins une surface de pose pour un pied humain (312, 314), de préférence deux surfaces de pose pour un pied humain (312, 314) chacune.

10. Système de traitement selon la revendication 9, dans lequel ladite au moins une surface de rangement (312, 314) a une surface d'au moins 50 cm² ou les deux surfaces de rangement (312, 314) ont chacune une surface d'au moins 50 cm².

11. Système de traitement selon la revendication 9 ou 10, dans lequel ladite au moins une surface de rangement (312, 314) comprend un revêtement (316, 318) de sorte que celui-ci présente un coefficient de frottement de glissement µ d'au moins 0,30, déterminé selon la norme DIN 51131.

12. Système de traitement selon l'une des revendications précédentes, dans lequel l'élément de base (300) comporte un élément d'éclairage (310), de préférence l'au moins une surface d'appui pour le pied humain (312, 314) de l'élément de base (300) peut être éclairée par l'élément d'éclairage (310).

13. Système de traitement selon l'une des revendications précédentes, dans lequel le système de traitement (100) comprend un appareil manuel de soin des ongles (600) relié à l'appareil de traitement (200) par un câble (602), notamment le câble (602) a une longueur d'au moins 10 cm.

14. Système de traitement selon l'une quelconque des revendications précédentes, dans lequel l'élément abrasif (402) présente une surface extérieure au moins partiellement cylindrique et la surface extérieure présente un diamètre d'au moins 20,0 mm, de préférence d'au moins 40,0 mm, de manière particulièrement préférée d'au moins 60,0 mm, de manière plus préférée d'au moins 80,0 mm, de manière la plus préférée d'au moins 100,0 mm.
